# EUROPEAN PATENT APPLICATION

(11) **EP 0 718 409 A2**
(43) Date of publication of application: **26.06.1996**
(21) Application number: 95120038.5
(22) Date of filing: 19.12.1995
(51) Int. Cl.: C12Q 1/68, C12N 15/10

(54) **DNA preparation method**

(30) Priority: 22.12.1994 JP 320288/94; 30.11.1995 JP 311950/95
(71) Applicant: HITACHI, LTD., Chiyoda-ku, Tokyo 101 (JP)
(72) Inventor: Kambara, Hideki, Hachiouji-shi, Tokyo 192 (JP); Okano, Kazunori, Shiki-shi, Saitama-ken 353 (JP); Fujimori, Kiyoshi, Shiki-shi, Saitama-ken 353 (JP)
(74) Representative: Strehl Schübel-Hopf Groening & Partner

(57) **Abstract**

A DNA preparation method is disclosed, said method comprising a first step of producing a plurality of DNA fragments having different lengths by digestion of double strand DNA; a second step of introducing into said DNA fragments the oligomer having a known base sequence at the portion digested in the first step by ligation; and a third step of carrying out complementary strand extension of the DNA fragments with primer having selective sequence on its 3'-terminus obtained in said second step and increasing the number of DNA fragment copies.

## Description

### BACKGROUND OF THE INVENTION

The present invention relates to the DNA analysis, particularly to effective long DNA analysis method.

According to prior arts, cloning has been used for long DNA analysis: Long DNAs are digested into fragments, and these fragment groups are introduced into such a vector as plasmid. Then they are infected onto Escherichia coli which are cultured on the culturing medium made of agar. In this case, colonies will be produced on the agar if they are cultured on the medium which allows survival of only the Escherichia coli containing the plasmid including the target DNA. The resulting Escherichia coli are of a single species constituting a colony where the Escherichia coli containing the plasmid containing the same DNA fragments are proliferated. The Escherichia coli are extracted from the single colony; they are cultured and purified, and the number of DNA fragment copies is increased. The prior art has been using the DNAs having been subjected to said cloning and culture process in order to determine base sequence.

### SUMMARY OF THE INVENTION

The prior art discussed above, however, requires a process of DNA cloning and Escherichia coli culturing in order to prepare a lot of template DNAs. This process is complicated and requires a lot of human labor, being unsuited to automation; it involves many problems to be solved if it is to be used for the analysis of such large-capacity DNAs as human genome.

The fluorescence detection type DNA sequencer (Nature 321, 674-679 (1986)) has been used for DNA base sequencing. This is based on a fluorescence detection of DNA fragments separated by gel electrophoresis. DNA fragments are produced by Sanger method (dideoxy method) through enzymatic strand extension reaction. The gel separation based method can read base sequence from 400 up to 700 bases. It is time-consuming and troublesome for sequencing DNA of several Kb to several tens of Kb because additional processes are required to prepare short template DNAs. One of the conventional method for that is a shotgun method (Anal. Biochem., 129, 216 (1983)). According to this method, the DNAs are digested randomly by ultrasonic wave to clone DNA fragments and insert them into Escherichia coli. After colonies have been cultured, Escherichia coli in each colony are cultured, thereby increasing the number of DNA copies. Then sample DNAs are extracted and analyzed. This method fails to identify what part of the sample DNA corresponds to the DNA contained in the extracted colony, before the sequence is determined. Besides, this method requires high redundancy in base sequencing. Usually it is necessary to extract and analyze the DNA fragments corresponding to 10 to 20 times of the target DNA strand length to be sequenced. Thus, much time and labor required in this approach are the big disadvantage of this method.

Furthermore, a method is also proposed to determine the DNA sequence one by one from the terminus (Primer walking; Proc. Natl. Acad. Sci. U. S. A. 86, 6917(1989)). According to this method, after a part of the DNA sequence has been determined, the next primer area is selected from the result, and the adjacent sequence is determined again by dideoxy method. This approach provides highly efficient sequencing, but it has a disadvantage that much time is required to determine the entire sequence in terms of time series, and that the primer must be prepared for every analysis, consuming much time and trouble.

Furthermore, a method is also proposed to use the restriction enzyme to produce short fragments of long DNAs and to determine the sequence of individual DNA fragments, with the results being connected. According to this method, the size of the fragments digested by restriction enzyme is uneven and cannot often be analyzed in one step. Besides, it has a disadvantage of requiring much time in knowing the connection among DNA fragments with one another. Thus, this method as in the above method is not suited to automation or large-scale DNA analysis.

The object of this invention is to propose a single DNA sample preparation method good for automation. The DNA sample is digested into fragments which are selectively amplified to be analyzed, using the primer having selective sequences of 1 to 4 bases at their 3'-termini to identify and select the DNA fragments, without DNA cloning or culturing of the Escherichia coli. This object of the present invention has been achieved successfully. The present invention provides a method of DNA sample preparation suited for automation, without DNA cloning and Escherichia coli culture process.

The present invention is based on the idea that any DNA fragment in a mixture containing a lot of DNA fragments can be separated by gel electrophoresis coupled with classification of terminal base sequences of the DNA fragments if the DNA sizes are lower than a few kb which can be successfully separated by polyacrylamide gel electrophoresis. Even if a mixture contains too many fragment species to separate by the gel electrophoresis, they can be separated by gel electrophoresis after classifying the fragments according to their terminal base sequences. Reversely, DNA fragments can be identified or individually separated by their terminal base sequences after being roughly separated by the gel electrophoresis.

The DNA preparation method in the present invention is comprised of at least three steps; a step of producing a plurality of DNA fragments by digestion, a step of introducing oligomers into the termini of fragments and a step of producing DNA fragment copies by complementary strand extensions with primers having selective sequences of 1-4 bases at their 3' termini.

As said double stranded DNA digestion (fragmentation) process, this approach allows the use of restriction enzyme process, exonuclease/S1 nuclease process, and physical digestion by laser or ultrasonic wave. Besides, said double stranded DNA digestion process also includes the process comprising the step of digesting and removing the loop-formed DNA single strand formed in the digestion (fragmentation) process using the S1 nuclease, and restoring the deficiencies of the double stranded DNA using the DNA polymerase and ligase.

Furthermore, the DNA preparation method according to the present invention comprises: (1) a step of generating a plurality of DNA fragments from double stranded DNA by digestion; (2) a step of introducing the oligomer having a known base sequence (first oligomer) into one DNA fragment terminus by ligation, and introducing the oligomer (second oligomer) having a known base sequence different from that of the first oligomer into another DNA fragment terminus; and (3) a step of carrying out complementary strand extension of said DNA fragment and increasing the number of DNA fragment copies, using the primer containing the base sequence complementary to the base sequence of said first oligomer, and the primer which includes the base sequence complementary to said second oligomer, fluorophore label being introduced into the 5'-terminus and which has selective sequences of 1 to 4 bases at their 3'-termini to identity and select the base sequence at one DNA fragment terminus.

Furthermore, the DNA preparation method according to the present invention comprises: (1) a step of introducing the oligomer having the known base sequence into the termini of DNA fragments produced by digestion of double stranded DNA; (2) a step of PCR (polymerase chain reaction) amplification of said DNA fragment, using at least one primer species which contains the base sequence complementary to that of the oligomer introduced to the fragment and which has a selective sequence less than 5 bases (1 to 4 bases) at the 3'-termini to identity and select one DNA fragment from the fragment mixture by their terminus sequences, and (3) a step of separating and fractionating the DNA fragment by gel electrophoresis.

Furthermore, DNA preparation method according to the present invention has: (1) a step of introducing the oligomer having the known base sequence into the DNA fragment group including double stranded DNA; and (2) a step of carrying out complementary strand extension at least once to increase the number of DNA fragment copies, using the primer which contains the base sequence complementary to that of this introduced oligomer and which has selective sequences less than 5 bases (1 to 4 bases) at their 3'-termini to identity and select the DNA fragment by its base sequence at terminus.

According to the present invention, the double stranded DNA is digested into fragments by restriction enzyme process, exonuclease/S1 nuclease process, and physical digestion by laser or ultrasonic wave, and the oligomer having the known base sequence is introduced into the terminus of this unknown DNA fragment to be changed into a form which allows amplification by PCR (polymerase chain reaction). That is, gel electrophoresis is used to approximately separate and fractionate the DNA fragments having a variety of lengths for each group. In another way, DNA fragments are digested by exonuclease or else at their termini into short fragments; the length is controlled by adjusting the reaction time, thereby getting the fragment groups having different lengths. The produced DNA fragments are modified through the ligation process introducing the oligomer having a known base sequence into the termini. In this case, the lengths of the DNA fragments within each fragment group are almost the same, but includes a variety of lengths in a strict sense of the word.

Said restriction enzyme includes HhaI, N1a3, Sau3A, TaqI, PstI, MaeI, HindIII, ECoRI. Exonuclease, S1 nuclease and ligase products are available on the market.

Then for the unknown DNA fragment with the terminus having a specified base sequence, complementary strand extension is repeated to increase the number of DNA fragment copies, using the primer which contains the base sequence complementary to that of this introduced oligomer and which has a selective sequence less than 5 bases at its 3'-termini to identity and select the DNA fragment through complementary strand extension reaction. This makes it possible to select and amplify one DNA group out of the total of four DNA fragment groups if the number of bases for selective sequence is one. The total fragment groups will be 16 if the number is 2, and 64 if the number is 3. This makes it possible to purify and prepare the DNA whose the terminus has a specific sequence. This can classify DNA fragments in a mixture according to their terminal base sequences.

The oligomer having the known base sequence to be introduced into the unknown DNA fragment terminus is 10 to 50 bases long, or preferably 18 to 30 bases long. When the oligomer length is smaller than 10 mer, stable hybridization of the primer on template DNA is not achieved, resulting in low PCR yield. On the other hand, synthesis of long oligomer is expensive and troublesome although it provides a stable hybrid with template DNA.

When the DNA fragments with a variety of lengths are separated and fractionated roughly by length by gel electrophoresis discussed above, the number of fragments in one group is reduced. This permits complementary strand extension reaction to increase the copy number of only one or two fragments having the specific base sequence at termini selected by the selective primer; this makes it possible to purify the DNA fragments by identifying and selecting the terminus base sequence. Moreover, repetition of complementary strand extension increases the number of the DNA fragment copies. They provides excellent DNA sequencing sample.

In another example, the DNA to be sequenced is digested by at least two types of 4-base recognition endonuclease to get the fragments having the size which can be sequenced in one sequencing operation. Different oligomers are introduced into each of these fragment termini. The first oligomer complementary to the first primer is introduced into the 3'-terminal of the DNA fragment produced by the first restriction enzyme, while the second oligomer is introduced into the 5' terminal of the DNA fragment produced by the second restriction enzyme. The first primer has a selective sequence comprising 2 bases at the 3'-terminus, and allows extension of the complementary strand of the fragment of the first fragment group whose 2 bases at the terminus are complementary. Use of heat cycle reaction causes extended strand to be hybridized with the fragment of the second fragment group having complementary sequence, resulting in longer strand. This produces an extended fragment group which has the first primer as 5'-terminus, and the sequence complementary to the second oligomer as 3'-terminus. These fragments are slightly overlapped with each other so that there can be obtained a fragment group which is convenient for determining the entire sequence. In addition, the DNA fragments can be separately prepared each by employing the first primer having the selective sequence and the second primer which has the same sequence as the second oligomer and has also the selective sequence, so that effective sequencing can be conducted.

The conventional long DNA sequencing depends on the shotgun method, causing the same sequence to be read 10 to 20 times on average repeatedly. Furthermore, it has to follow a troublesome step of cloning and Escherichia coli culturing, and is not suited for automation.

The present invention, by contrast, permits the amplification of one DNA fragment in the fragment groups separated by gel electrophoresis to contain the DNA fragments having almost the same length by terminal sequence selective PCR, thereby obtaining the orderly arranged DNA fragment groups whose lengths are changed at almost the same rate. In these fragments, the termini on one side of these DNA groups start at the same site, and the end positions on the other terminus change at intervals of specified lengths. The termini are sequenced successively starting from the longest DNA chain. This method ensures effective determination of all the sequences by continuing to read the sequences information until overlap occurs with the sequences close to the termini of the shorter DNA fragments.

The lengths of DNA fragments are arranged so that the portion of overlapped sequence between fragments may be less than 50 base long; this is equivalent to about 10 to 20 percent of the total sequence. Even both DNA double strands are sequenced, the overall redundancy is 2.5 at most, amounting to one fourth to one eighth that of the conventional method. The present invention has no problem of small DNA fragments falling off, without fragments being bound together, thereby failing to determine the overall sequences, although these problems are frequently found in the conventional method using the cloning step. Furthermore, it has an advantage of facilitating automation due to the absence of the cloning step.

The method using two restriction enzymes provides more effective DNA sequencing. The first and second fragment groups are produced by the first and the second enzymatic digestion of DNA, respectively. They are digested at different sites because different restriction enzyme are used. The 3'-terminus of fragment in the first group always appears in a fragment belonging to the second group. The 3'-terminus can hybridize on the fragment in the second group to extend its strand to make a longer fragment. Complementary strand extension carried out using the first primer to be hybridized with the 3'-terminus of the DNA fragment on the first fragment group will produce complementary strands up to the 5'-terminus of this DNA fragment. The sequence of this complementary strand on the 3'-terminus side is also complementary with the DNA fragment in the second fragment group. The temperature of the reaction mix containing extended DNA strand discussed above is raised to cause thermal dissociation of the hydride. Then the temperature is lowered to produce the hydride, and the hybrid is formed between the extended complementary strand and the DNA in the second fragment group. Said extended strand is further extended by complementary strand extension, resulting in complementary strands up to the 5'-terminus of the DNA in the second fragment group to be synthesized. Namely, this produces fragments comprising a combination of the first and second fragments. Complementary strand extension using the primer to be hybridized with this 3'-terminus produces a template with the 5'-terminus of the DNA fragment of the second group appended to the 5'-terminus of the first fragment. Sequencing reaction using the first primer identifies the sequence of the DNA group pertaining to the first group and part of the DNA fragment adjacent thereto, making it possible to identify the linkage of each DNA fragments using the sequence overlap.

According to the present invention, as discussed above, oligomer is introduced into the DNA fragment terminus. Using the primer having selective sequences of 1 to 4 bases at their 3'-termini to identify and select the base sequence on one DNA terminus, the primer being hybridized with said oligomer portion, PCR amplification is carried out, thereby selecting only the specific fragments. This procedure eliminates the need of a cloning step, ensuring a fully automated sample adjustment.

According to the present invention using 2 bases as the selective sequence, furthermore, DNA is digested by the first restriction enzyme to produce the small fragment groups which are sequenced in parallel using sixteen (4x4=16) species of primers prepared separately. Moreover, prior to sequencing, the sequencing template is prepared with the PCR after adding the DNA fragments obtained by digestion with the second restriction enzyme, thereby obtaining the template DNA by extending the DNA of the first DNA fragment group to the terminus of the DNA pertaining to the second DNA fragment group, and producing a fragment group covering the entire length of the DNA while overlapping. The sequence information obtained from this template DNA contains the information on overlap with the adjacent DNA fragment. This provides an easy determination of the overall base sequence.

### BRIEF DESCRIPTION OF THE DRAWINGS

Fig. 1 is a conceptual drawing illustrating an example of long DNA sequencing according to the present invention;
Fig. 2 is a conceptual drawing illustrating an example of obtaining the DNA fragment by using enzyme according to the present invention;
Fig. 3 is a drawing representing the relationship between the primer used in the present invention and the oligomer introduced into the DNA fragment;
Fig. 4 is a drawing illustrating the method of obtaining the DNA fragments having a specified length by selective DNA polymerase reaction from the DNA fragments separated by gel electrophoresis and having almost the same lengths according to the present invention;
Fig. 5 is a conceptual drawing illustrating the purification method where a DNA fragment group contains the DNA fragments having a same sequence and little bit different lengths according to the present invention;
Fig. 6 is a conceptual drawing illustrating a method where selective PCR after physically cutting the DNA is employed to produce DNA fragments according to the present invention;
Fig. 7 is a conceptual drawing illustrating a method of using two species of restriction enzymes for producing DNA fragments and of making extended fragments for overlap analysis of the fragments to obtain total sequence according to the present invention;
Fig. 8 is DNA fragment spectra obtained by polymerase reaction with 16 selective primers and digested products of PUC DNA, respectively;
Fig. 9 is a conceptual drawing illustrating a method to classify DNA fragments into 256 groups with two types of 16 selective primers using PCR amplification; and
Fig. 10 is a conceptual drawing illustrating a method to prepare overlapped DNA fragments covering the entire DNA.

### DESCRIPTION OF THE PREFERRED EMBODIMENTS

The following gives concrete description of the present invention with reference to embodiments, without the present invention being restricted to the embodiments illustrated below:

### First Embodiment

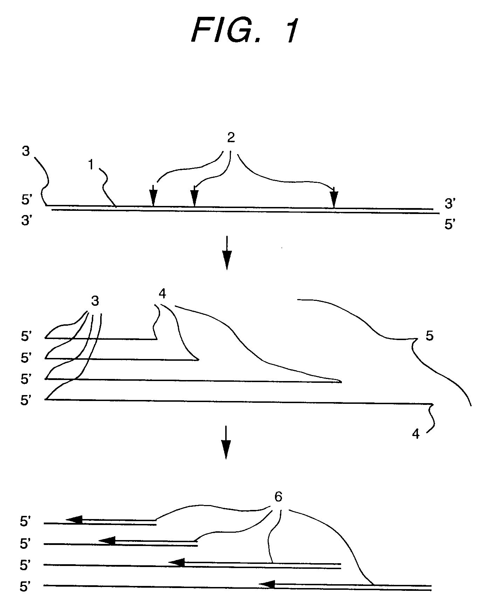

As illustrated in Fig. 1, analysis of a long DNA requires chemical or physical cutting of the sample DNA1 at positions 2. This is followed by the step of preparing from these cut fragments a single strand DNA fragment wherein one DNA terminus 3 comprises a specified terminus base and the other terminus 4 is cut at various positions of sample DNA1. The sequence is successively read from the 3'-terminus (terminus 4) to get the portion being sequenced 6.

The first embodiment refers to the case of using the DNA fragment obtained by terminus digestion.

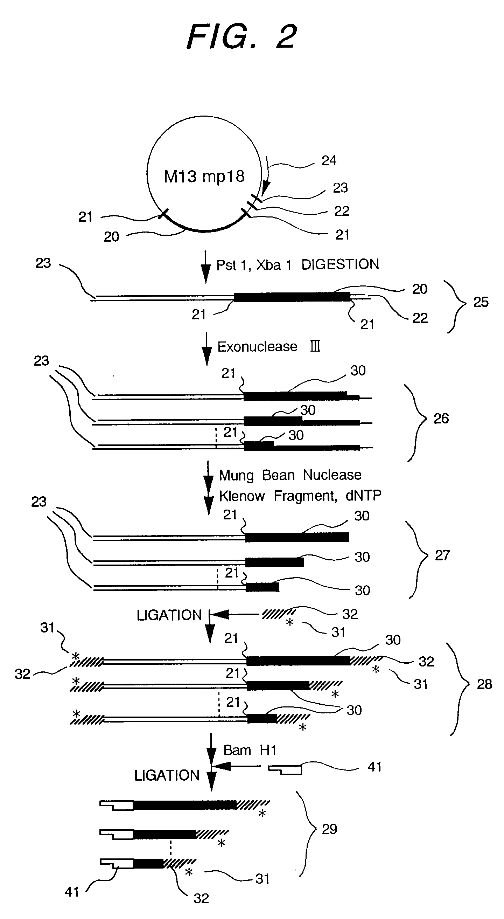

One of the methods of producing the DNA fragments as shown in Fig. 1 is a successive digestion of the terminus bases by a combination of exonuclease (by Amersham Co.) and S1 nuclease (also by Amersham Co.). The following shows the case of sequencing a long DNA (about 10 kbp long) inserted into a vector. The double stranded plasmid DNA of M13 phage such as M13mp18 (by Amersham Co.) is used as a vector. Fig. 2 illustrates the process of sequencing sample preparation according to the present invention using the double strand M13mp18. The DNA20 to be sequenced is cloned to the restriction endonuclease Bam H1 site 21. This vector has the cutting sites of Xba122 and PST123 between the Bam site 21 and priming site 24. When cut by restriction enzyme, a long DNA fragment 25 is obtained, as shown in Fig. 2.

Then exonuclease III (by Amersham Co.) is made to act on the long DNA fragment 25 for several minutes. Exonuclease III digests the double strand DNA having the 5'-cohesive end and blunt end successively starting from the 3'-terminus. By changing the reaction time, it is possible to get, as products, DNA fragment groups 26 wherein double stranded portion 30 in Fig. 2 has different lengths 26. This is followed by the step of causing Mung beam nuclease (S1 nuclease), nuclease (by Amersham Co.) and Klenow fragment (DNA pomerase) (by Amersham Co.) to act on the DNA fragment group 26, thereby obtaining the DNA fragment group with blunt end 27 by removing the single strand portion. DNA fragment group 28 is obtained by ligating the oligomer with known base sequence 32 having a cohesive end and blunt end to DNA fragment group 27. To prevent the oligomer 32 from being connected to the DNA fragment 27 in the opposite direction, the non-hybridized end of the oligomer 32 has a cohesive end (not a blunt end). Fluorophore 31 is bound to the 5'-terminus of non-hybridized end (cohesive end) of the oligomer 32 for monitoring at the time of fractionation of the DNA fragment. Ligation reaction is carried out for 6 to 12 hours.

Then the DNA fragment group 28 is digested by the restriction enzyme Bam HI and the oligomer with known base sequence 41 (one terminus having a cohesive end) is ligated to the cut site, thereby gaining the DNA fragment group 29. The base sequence of the oligomer 41 ligated to the Bam HI site is made different from that of the oligomer 32 hybridized to the blunt end. The Bam HI site side (to which the oligomer 41 is to be hybridized) of this DNA fragment 29 is cut at the specified site and connected with the oligomer. However, since the digestion of the other side is due to deletion, the oligomer (32) ligated to the other side is not bound to the fragment digested at specified sites in a strict sense of the word. One group 29 comprises a mixture of the DNA fragments having many different lengths. That is, the position of the side where the oligomer 32 of the DNA fragment 29 (blunt terminus) is not specified in a strict sense.

DNA sequence reaction is carried out from the side of oligomer 32. When DNA sequencing is carried out for one group 29 comprising a mixture of the DNA fragments having many different lengths, signals comprising a mixture of various sequences are obtained, failing to achieve successful sequencing. This makes it necessary to select only the DNA fragments having a specified length from the mixtures, thereby determining the base sequence. According to the prior art, these fragments are again cloned (subcloned) and specified colonies are gained after colony culturing. By re-culturing them, the DNA fragments have been purified and the number of copies has been increased. According to the present invention, only the specified DNA is amplified by selective PCR, instead of the conventional subcloning. The portion following the oligomer 32 of the DNA fragment 29 has base sequences different for each fragment species. If 2 bases on the 3'-terminus side of the primer are completely hybridized in DNA complementary strand extension, complementary strand extension takes place; however, if not, complementary strand extension does not take place or takes place to a very small extent. Thus, the inventors have selected and amplified only the specific DNA strands, taking advantage of the fact that the two bases following the hybridized oligomer 32 are different for each species of the fragments (there can be fragments of different species having the same sequence of two bases if there are many species of fragments).

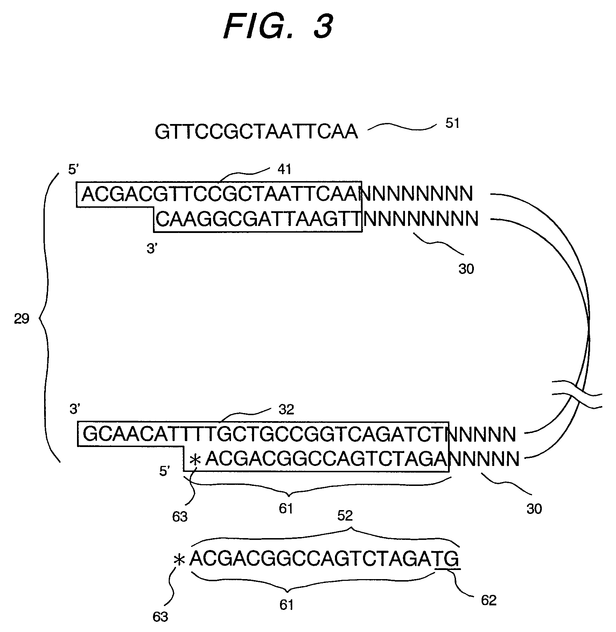

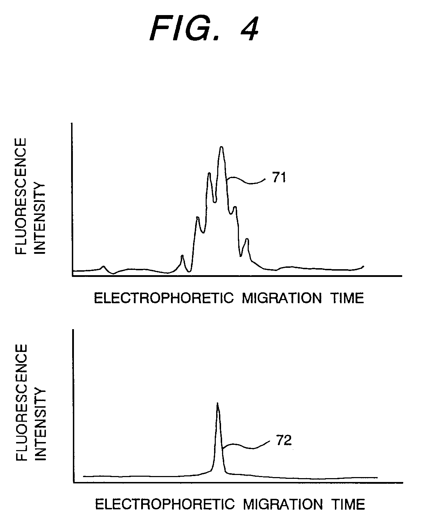

Fig. 3 illustrates the relationship between the primer used for amplification and the fragment where oligomer is introduced on both termini. The N represents either A, T, G or C. The primer used for amplification includes (1) primer 51 hybridized with oligomer 41, (2) primer 61 hybridized with oligomer 32 having the 5'-terminus labeled with fluorophore, and (3) primer 52 having sequence TG62 following it. It should be noted that the sequence TG is a selective sequence used to select the DNA fragment species conforming to the type of the base sequence at the 5'-terminus. Accordingly, when the selective sequence comprises two bases, there are 15 species of selective sequences in addition to sequence TG, providing selective amplification of the DNA fragment species having these 15 species of base sequences at 5'-terminus. Namely, in this case, each of a total of 16 species of primers is composed of the selective sequence portion comprising two bases and the portion of oligomer 61, the portion of oligomer 61 forming a common sequence. Primer 51 hybridizes commonly to all fragments. On the other hand, complementary strand extension with the primer 52 completely hybridized is effectively implemented in the case of the DNA fragment where the terminus has a sequence of AC following the common sequence. Only the specific species out of a plurality of fragment species having different lengths could be amplified by PCR with a selective primer. This will be illustrated with reference to Fig. 4. One fraction of the DNA fragment group before PCR contains DNA fragments having different lengths. The upper sketch in Fig. 4 shows the distribution of the number of their copies (electrophoresis pattern) 71. If a mixed DNA fragment group containing DNA fragments with different lengths, for example, is amplified by the primer having the terminus sequence of TG, then only the DNA fragments with terminus sequence of AC out of the fragments obtained from the sample DNAs of said mixed DNA fragment group is subjected to selective amplification. The electrophoresis pattern of the DNA fragments after PCR can be represented as 72 in the lower sketch of Fig. 4. This provides selective amplification and extraction of one specific species of DNA fragments of the mixed DNA fragment group.

The above has shown the way of obtaining the fragments by amplification of only the specific species of fragments in the terminus digestion products (deletion products) by enzyme (exonuclease). By changing the terminus digestion reaction time, it is possible to get the DNA fragments having different, but almost the same, lengths in conformity to the terminus digestion reaction time. By processing these DNA fragment group in said manner, it is possible to amplify only a specific fragment species in each group in conformity to the terminus digestion reaction time and to provide the same length for each group. Sequential analysis in this manner permits long DNA sequencing.

### Second Embodiment

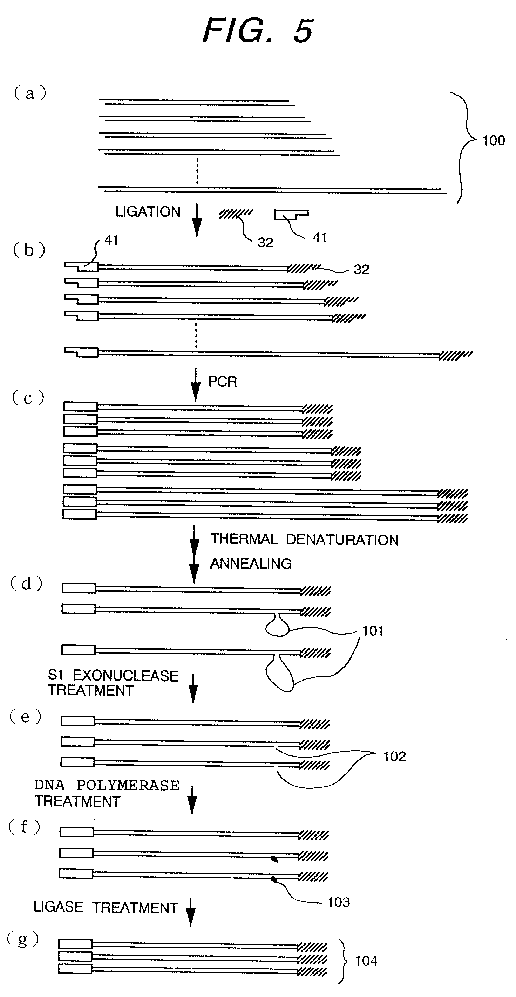

If DNA fragments having a plurality of lengths are still included in the DNA fragment group obtained in the manner of amplifying said specified species of DNA fragments, they can be purified according to the following method: Fig. 5 gives the outline of the method. One group (a) includes DNA fragment group 100 having different lengths produced by deletion. Known oligomers 41 and 32 are hybridized to both termini of said DNA fragment, as shown in group (b). They are then subjected to PCR amplification. (For the primer in PCR, see the description with reference to Fig. 3.) In this case, the primer 52 hybridized with the oligomer 32 has two bases 62 for base selection (herein as OCR) at the common sequence 61 and its 3'-terminus; thus, of various fragments, only the fragments exhibiting a complete agreement of the TG portions are amplified by PCR. This results in three species of fragments being produced, as shown in group (c). After the primer is removed, they are mixed and are subjected to thermal denaturing and annealing; then DNA pairs having different lengths are produced in addition to the DNA pairs having the same length, as shown in group (d).

Since the base sequence at both termini of these DNAs is common, a loop 101 is formed on the way. If this single stranded portion is digested by SI nuclease, it is turned into the type having a cut 102 on part of the double strand, as shown in group (e). If there is a lack of the cut portion by one base or more, ligase repair reaction to be implemented later will not take place smoothly. To prevent this, DNA polymerase is made to act on the cut portion as in 103, and group (f) is obtained by complementary strand extension. This is followed by the step of repairing the double strand by DNA ligase to get group (g). Repairing the double strand by the DNA polymerase and DNA ligase will result in higher percentage of the short DNA strands having the same length (double strands) 104. Then the DNA strands (double strands) 104 are thermally denatured to get single strands, and the reaction of getting the group (g) from said group (c) is repeated several times; this makes it possible to turn a plurality of species of DNA strands having different lengths into the fragments having more uniform lengths, as the DNA strands (double strands) 104. Thus, it is also possible to make uniform the length of one fragment group gained by deletion. The process of using the SI nuclease may be used for group (a) before ligation of the oligomer.

### Third Embodiment

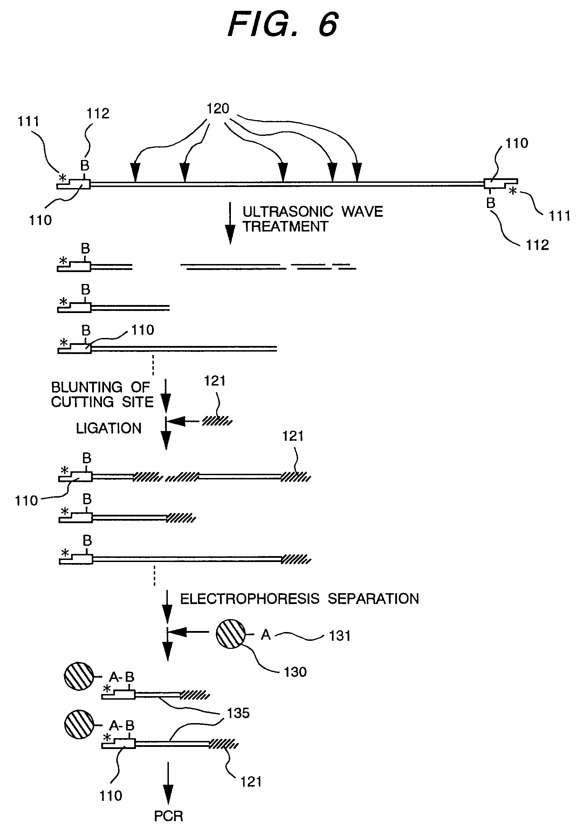

In said embodiment 2, the DNA is reduced in size by deletion. This applies also to the fragments cut by ultrasonic wave or laser or chemical digestion. By way of an example, Fig. 6 shows the case of using ultrasonic wave for cutting. When ultrasonic wave is used, cutting takes place at random, and cut site 120 is arandom site. The fragments are produced is randomly from various parts of the DNA. To improve analysis efficiency, it is more convenient to get DNA fragments having different lengths, starting from a specific site.

The oligomer 110 bonded with fluorescent label 111 and biotin 112 are ligated on both termini of the sample DNA prior to digestion. Then the digestion of the DNA and the ligation of oligomer 121 to the fragments are carried out. The fluorophore labeled side of the fragment is protected against ligation reaction.

For example, the fluorescent labeled terminus of the fragment is formed into a cohesive terminus to prevent the ligation of oligomer to the terminus. The sample DNA containing label is made into fragments, and the digested portion is made blunt; then the oligomer 121 having the base sequence different from that of oligomer 110 is bound thereto by ligation. The generated fragment is fractionated by electrophoresis according to size. The magnetic bead 130 holding the streptavidin 131 is used to extract only the separate DNA fragments 135 having more similar lengths to which the biotin is bound ; then PCR amplification is carried out using the primer complementary to oligomer 110 and oligomer 121. In this case, the primer hybridized with the oligomer 121 must be the primer which has the base separation sequences, for example, those with TG on the 3'-terminus side, in addition to complementary sequence, as in the case of Fig. 5. This allows only the fragments having specific lengths to be selected from the DNA fragment group having similar lengths, and to be amplified. This has made it possible to get 1 to 3 DNA fragments for each group fractionated according to the size. The method according to Embodiment 2 can be used to make the length uniform for the group containing a plurality of DNA fragments having different lengths.

According to sample preparation discussed above, a DNA fragment group having a specified interval of length can be obtained from the sample DNA terminus. Thus, successive base sequencing of these DNA fragment groups ensures effective DNA sequencing. The DNA fragments pertaining to each DNA fragment group should preferably be adjusted to be about 300 base long. Furthermore, two groups having similar lengths out of the DNA fragment groups should be prepared to have the difference in lengths of about 3000 bases. This is because the length allowing easy reading of the sequence by the DNA sequencer is about 400 bases. When using a long gel (capable of determining 800 to 1000 base sequences) capable of determining the sequence of longer DNA fragments, DNA fragments may be prepared to have a difference in length of 600 to 800 bases.

### Fourth Embodiment

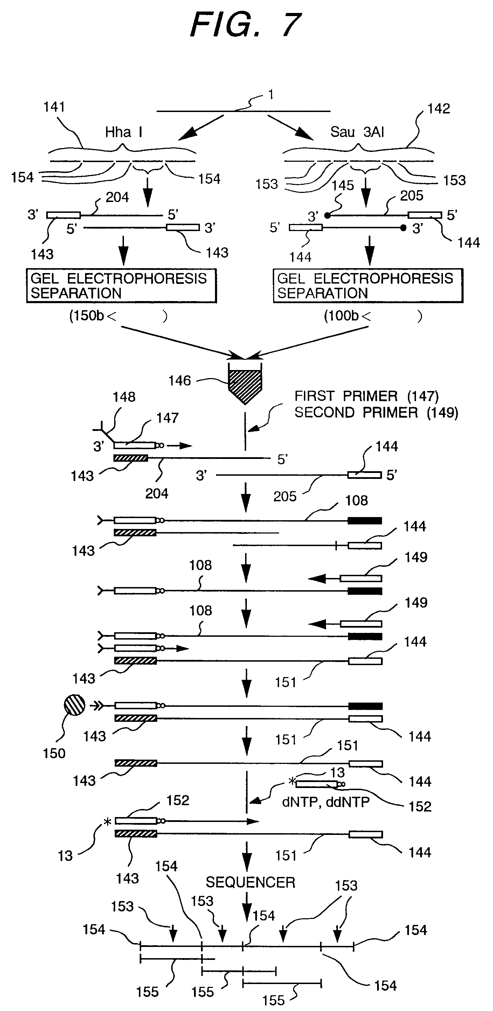

The fourth embodiment is concerned with the method of digesting the DNA to be analyzed by restriction enzyme to determine the sequence of each fragment and that of the adjacent DNA fragment and to bind fragments using the sequence overlapping, thereby determining the entire DNA sequence. The enzymatic digestion products should cover all the sequence in a DNA without overlap. If we select long fragments among the products and determine their sequences together with the adjacent sequences to overlap each other, the most efficient sequencing can be realized. Because it will give low redundancy in determined sequence and all the fragments to be sequenced can be sequenced in parallel. The following describes an example of using pUCDNA (approx. 2.7 k bases): Fig. 7 illustrates the flow of the present invention.

Hhal (GCG↓C) was utilized as the first restriction enzyme, while Sau3A1 (↓GATC) was used as the second restriction enzyme. Needless to say, other restriction enzymes such as N1a3, PssI, MaeII, HinPl, TaqI, MseI, Sau96, and other ends nuclease may be used. The sample DNA is dissolved in buffer solution and is dispensed in two vessels. Hhal and Sau3A1 are put in each of the vessels to digest the DNA. The enzyme is removed from the solution containing the first segment group digested by Hhal, and the first oligomer having the known sequence is ligated to the 3'-terminus. (The terminal transferase may be used, and poly-A tail, etc. may be added.) This oligomer is used as a priming portion, so 10 mer or more is required. Nothing is ligated to the 5'-terminus.

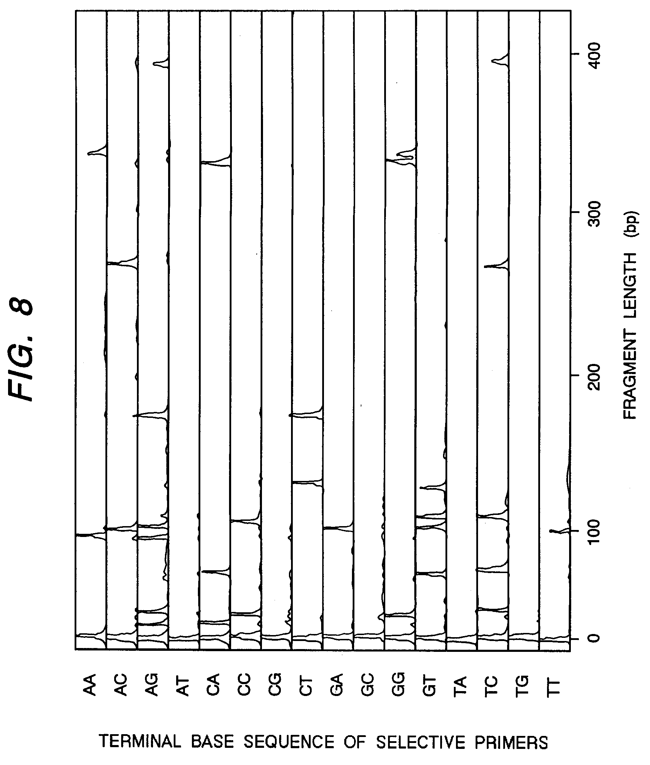

The second oligomer (10 mer or more) of the known sequence is ligated to the 5'-terminus side of the second fragment group digested by Sau3A1. No oligomer is ligated to the 3'-terminus, which is blocked by introduction of dideoxynucleotide (terminator) by DNA polymerase or other means; namely, means should be taken not to allow the primer of the known sequence to be hybridized to this 3'-terminus side even after repeated polymerase reactions. This is because, if the oligomer complementary to the oligomer introduced to the 5'-terminus is introduced into the 3'-terminus, the fragment pertaining to the second fragment group will be subjected to PCR amplification due to cyclic complementary chain extending reaction using the second oligomer; this must be avoided because the purpose is to make the extended fragments as stated below. A part of the sample solution containing the first fragment group is used to check fragment sizes in the sample by gel electrophoresis of the complementary strand extension products using the 16 primers (first primers) which are hybridized with the unknown sequence portions of the DNA fragments at the 3'-termini --- the 16 species of fluorescent label primers (the first primer having the selective sequence) which have the selective sequence comprising two bases, thereby obtaining the gel electropherogram (spectrum 1 in Fig. 8). This indicates the length of the DNA fragment for each of the two-base sequences at the terminus in the first segment group. As several primers produced more than one products after polymerase reactions, the DNA sample (fragments mixture) is separated by size prior to sequencing.

The first fragment group is subjected to gel electrophoresis separation, and fractionation of DNA fragments by size is carried out by making reference to spectrum 1 to ensure that about one DNA strand is contained for each group separated by two bases at the terminus. Small fragments may be excluded. In the example of pUC, fractionation was in the groups of two sizes; about 150 to 270 bases and 300 to 500 bases (first and second groups). On the other hand, the second fragment group was also subjected to gel electrophoresis separation to carry out fractionation of fragments longer than about 100 bases. The DNA fragment contained in the second fragment group is added to the first and second groups comprising the first fragment group.

The thermostable DNA polymerase, polymerase reaction substrate, biotin label first primer and primer having virtually the same sequence as the second oligomer are added to the first and second groups, thereby carrying out PCR amplification. This allows the DNA of the first fragment group to amplify only the DNA fragments which are extended to the 5'-terminus of the DNA pertaining to the second segment group partly having the same sequence. After the amplification, only the extended fragments are extracted using the bondage between biotin and avidin, and is utilized as a template for DNA sequence reaction. After amplification, the double strand undergoes thermal dissociation to provide the template DNA strand having the site where the first primer hybridizes. The solution containing this template DNA strand is divided into fractions according to the number of DNA fragments contained in the first group, and the first primer having the selective sequence is added to each, resulting in sequencing reaction.

Use of the extended first fragment group as a template allows the first DNA fragment sequence and its adjacent sequence to be obtained. The adjacent sequence can be used to get the entire sequence by bonding DNA fragments.

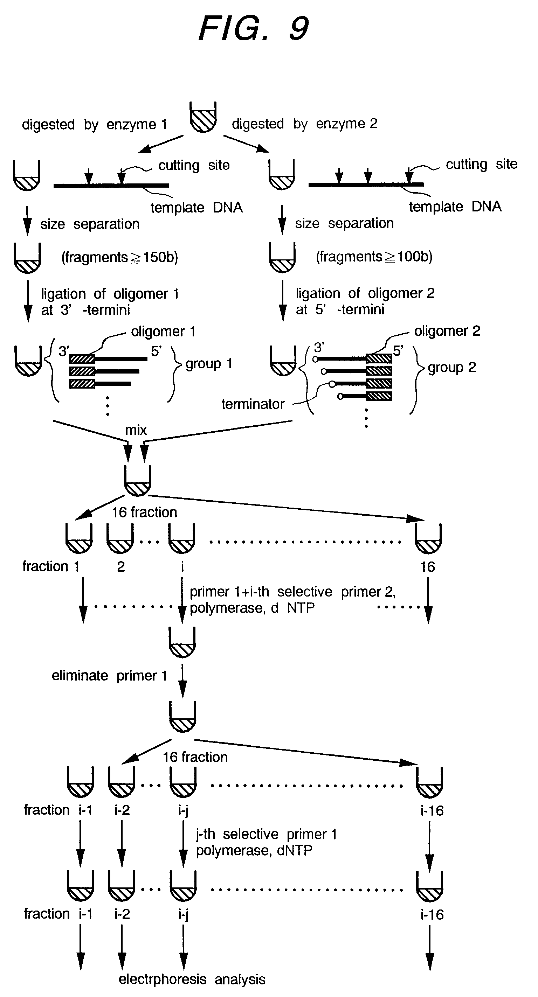

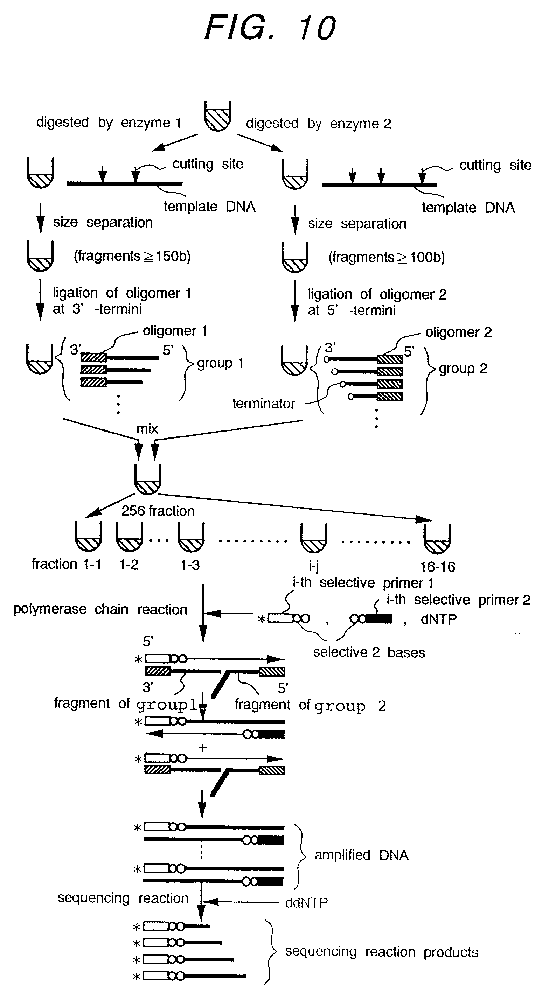

This method will be more effective if the extended fragments are produced by PCR using the primer hybridized to each of the fragments digested by two species of restriction enzyme (as shown in Figs. 9 and 10).

A half of the DNA is dispensed and is cut by the first 4-base recognition enzyme Hhal to prepare the first fragment group. Then the first oligomer is ligated with the known sequence at the 3'-terminus. Another half of the DNA is dispensed into another vessel and is cut by the second enzyme Sau 3Al to prepare the second fragment group. Then the second oligomer is ligated with the known sequence at the 5'-terminus (Fig. 9). The 3'-terminus is blocked to prevent it from extending at the time of complementary strand extension. The first fragment group is subjected to gel electrophoresis to collect the DNA fragments which are over 150 bases long. The second fragments are separated to collect fragments over 100 bases long. The collected second fragments are mixed with said first fragment group. The mixture solution is divided into 16 fractions (1 to 16 groups), and is subjected to PCR amplification. This step uses the first primer which is virtually complementary to the first oligomer, and the sixteen species of the second primers which have the sequence common to the second oligomer and which have a part of the restriction enzyme recognition sequence and the selective sequence comprising two bases on the 3'-terminus side. In the reaction, one species of selective primer and the first primer are introduced into each group. This will result in the first primer undergoing complementary strand extension up to the strand which is complementary to the secondary oligomer. This is carried out through cycle reactions by rehybridizing the complementary strand to the fragment pertaining to the second fragment group. Of these extended strands, only the strands with which the second primer having the selective sequence is hybridized are subjected to PCR amplification, thereby producing a great many copies. Namely, the DNA is classified according to the two-base sequence following to the second oligomer and the cutting site sequence at the 5'-terminus and the classified DNA is amplified. Primers used in PCR reactions are removed from reaction products of sixteen groups obtained in said manner, and products in each group are divided into 16 parts as a total of 256 fractions. Each species of the first primers having fluorophore taggs and the selective sequences (16 species) each comprising two bases at the 3'-terminus is added to each fraction, and complementary strand extension is carried out. DNA fragments contained in each fraction (a total of 256 fractions) are classified according to the two-base sequence following to the known sequence of the first oligomer and a part of recognition sequence on the 3'-terminus side of the fragment (on the 5'-terminus side when viewed from primer extension strand after PCR reactions). Gel electrophoresis is carried out to check the extended DNA stands in a total of 256 (16 x 16). When there is only one fragment in the fraction, the fraction contains only one species of DNA fragment with which the selective primers hybridized to be extended by complementary strand extension; so dideoxynucleotide (ddNTP) is added thereto for sequencing reaction, and the sequence is determined by the fluorescent DNA sequencer. When two or more species of DNA fragments have been included in the fraction, the sequence is determined after size separation by gel electrophoresis or it is determined by the primer having a selective sequence of 3 to 4 bases.

### Fifth Embodiment

This example discloses a method of getting DNA fragment groups containing different DNA fragments by utilizing PCR (Fig. 10).

DNA is dispensed and is digested by four-base recognition enzyme Nla3 (CATG↓). Over about 150 bases long DNA fragments are fractionated by the gel electrophoresis, and are bound to the first oligomer having the known sequence on the 3'-terminus side of the digested fragment. The unreacted oligomer and ligase are removed and the first DNA fragment group is obtained after purification. DNA is dispensed and is digested by the second 4-base recognition enzyme Sau3A1. Over 100 bases long DNA fragments are fractionated by the gel electrophoresis, and are bound to the second oligomer having the known sequence on the 5'-terminus side of the fragment. The ligase and similar substances are removed and the second DNA fragment group is obtained after purification.

Both fragment groups are mixed in equal mols as shown in Fig. 10. The mixed samples are dispensed into 256 tubes to be used as PCR templates. Sixteen species of the first primers which contain the sequence virtually complementary to the first oligomer and which have the selective sequence of two bases on the 3'-terminus side are prepared, while sixteen species of the second primers which contain the sequence virtually equal to the second oligomer and which have the selective sequence of two bases on the 3'-terminus side are prepared as the second primer. In this case, biotin or similar substance is put close to the 5'-terminus of the second primer. A total of 256 primer pairs resulting from all possible combinations between the first and second primers are prepared, and each pair is added to each of the mixed samples. Thermostable DNA polymerase and DNA complementary strand extension substrate are added to the samples to carry out PCR amplification.

Various types of DNAs are present in the mixed sample. The primer 1 having the selective sequence perfectly matching to the complementary strand in the first DNA fragment group is extended first. Then the extended primer 1 is hybridized with the DNA fragments in the second DNA fragment group which have a complementary sequence to the extension strand, resulting in strands being extended. The primer 1 extension stops after extension up to the sequence complementary to the second oligomer located at the 5'-terminus of the second fragment group. This DNA fragment is referred to as hybrid fragment (a bound form of the DNA fragments pertaining to the first and second fragment groups). The second primer can be hybridized with the 3'-terminus of this strand extended from primer 1; however, the second primer can extend only when the selective sequence located on the 3'-terminus of the second primer is matched. Namely, complementary strand extension is carried out only when matching is found with the DNA fragment pertaining to each of the first and second fragment groups where the selective sequences of the first and second primers serve as templates; then PCR amplification is carried out. Namely, only the hybrid fragments matched to the selective sequences of the first and second primers are subjected to selective amplification. After amplification, DNA is purified or the biotin in the second primer is trapped by the avidin fixed on the surface of such solid as bead (biotin/avidin binding), thereby extracting only the target DNA.

The following step of determining the sequence by DNA sequencing reaction is the same as before, as given in the said examples. In the present embodiment, DNA was dispensed into 256 tubes. It is also possible to identify the first or second primer for each species on the surface of a solid and to immobilize it there, with each of the remaining species of primers added separately; then reaction may be carried out.

The following lists up the reference numerals in the drawings with descriptions:
- 1, 20:: sample DNA
- 2:: cutting cite
- 3:: one DNA fragment terminus
- 4:: other DNA fragment terminus
- 5:: DNA fragment group
- 6:: portion being sequenced
- 21:: Bam HI site
- 22:: Xba I site
- 23:: Pst I site
- 24:: priming site
- 25:: long DNA fragment
- 26:: DNA fragment being degradated by Exonuclease III
- 27:: DNA fragment with blunt teminus
- 28:: DNA fragment having oligomer with known base sequence at both termini
- 29:: DNA fragment having oligomers with different known base sequences at each terminus
- 30:: double stranded portion
- 31, 63, 111:: fluorophore
- 32:: oligomer with known base sequence
- 41:: other oligomer with known base sequence
- 51:: primer being hybridized with oligomer on the Bam HI site
- 52:: primer hybridized on the oligomer 32
- 61:: common base sequence portion
- 62:: two bases adjacent to common base sequence portion
- 71:: electrophresis pattern of DNA fragments before PCR
- 72:: electrophresis pattern of DNA fragments after PCR
- 100:: DNA fragments with different lengths produced by deletion
- 101:: non-hybidized portion produced in processes of denaturing and annealing DNA fragments
- 102:: gap portion produced by S1 nuclease decomposition
- 103:: extended complementary strand portion at the gap portion
- 104:: double strands with the same length
- 110:: oligomer introduced into DNA by ligation
- 112:: biotin
- 120:: cutting site produced by ultrasonic wave decomposition
- 121:: other oligomer
- 130:: magnetic beads
- 131:: streptavidin
- 135:: separated DNA fragment with nearly same length
- 204, 141:: DNA fragments produced by digestion with enzyme 1
- 205, 142:: DNA fragments produced by digestion with enzyme 2
- 143:: the first oligomer
- 144:: the second oligomer
- 145:: blocked 3'-terminus
- 146:: fragment mix
- 147:: the first primer having selective sequence
- 148:: biotin
- 149:: the second primer
- 150:: avidin covered particle (magnetic heads)
- 151:: template DNA fragment for sequencing
- 152:: fluorophore labeled first primer having selective sequence
- 153:: cutting sites for enzyme 2
- 154:: cutting sites for enzyme 1
- 108, 155:: produced extended fragment
- 13:: fluorophore

## Claims

1. A DNA preparation method comprising:
(1) a first step of producing a plurality of DNA fragments having different lengths by digestion of double stranded DNA ;
(2) a second step of introducing into said DNA fragments an oligomer having a known base sequence by ligation at the portion digested in the first step; and
(3) a third step of carrying out complementary strand extension of the DNA fragments obtained in said second step and increasing the number of DNA fragment copies, using a primer which contains the base sequence complementary to said oligomer and which has selective sequences of 1 to 4 bases at the 3'-terminus to identity and select the base sequence at one DNA fragment terminus.

2. A DNA preparation method according to Claim 1 wherein said first step contains the step of restriction enzyme treatment.

3. A DNA preparation method according to any of the preceding claims wherein said first step contains the step of exonuclease and Sl nuclease treatment.

4. A DNA preparation method according to any of the preceding claims wherein said first step contains the step of using Sl nuclease to digest a single strand DNA portion, and using DNA polymerase and ligase to repair the double strand DNA.

5. A DNA preparation method according to any of the preceding claims wherein said first step contains the step of physical cutting by laser.

6. A DNA preparation method according to any of the preceding claims wherein said first step contains the step of physical cutting by ultrasonic wave.

7. A DNA preparation method comprising:
(1) a first step of producing a plurality of DNA fragments having different lengths by digestion of double stranded DNA;
(2) a second step of introducing into one terminus of said DNA fragment a first oligomer having a known base sequence at the portion digested in said step 1 by ligation, and introducing a second oligomer having a known base sequence different from that of the first oligomer into the other terminus of said DNA fragment; and
(3) a third step of carrying out complementary strand extension of the DNA fragments obtained in said second step and increasing the number of DNA fragment copies, using a primer containing a base sequence complementary to the base sequence of said first oligomer, and a primer which includes a base sequence complementary to said second oligomer, fluorophore label being introduced into the 5'-terminus and which has selective sequences of 1 to 4 bases at the 3'-terminus to identity and select the base sequence at one terminus of said DNA fragment.

8. A DNA preparation method comprising:
(1) a first step of obtaining the DNA fragments having a known base sequence introduced into the digested portion produced by digestion of the double stranded DNA;
(2) a second step of PCR (polymerase chain reaction) amplification of said DNA fragment, using at least one species of the primer which contains the base sequence complementary to that of said oligomer and which has selective sequences of 1 to 4 bases at the 3'-terminus to identity and select the base sequence at one terminus of said DNA fragment; and
(3) a third step of separating and fractionating the DNA fragment by gel electrophoresis.

9. A DNA preparation method comprising:
(1) a first step of obtaining the DNA fragment with an oligomer having a known base sequence introduced into the DNA fragment group including a plurality of double stranded DNA; and
(2) a second step of carrying out complementary strand extension at least once to increase the number of DNA fragment copies, using a primer which contains a base sequence complementary to that of said oligomer and which has selective sequences of 1 to 4 bases at the 3'-terminus to identity and select the base sequence at one terminus of the DNA fragment.

10. A DNA preparation method comprising:
(1) a first step of obtaining the DNA fragment with an oligomer having a known base sequence introduced into the DNA fragment group including a plurality of double stranded DNA; and
(2) a second step of carrying out complementary strand extension at least once to increase the number of DNA fragment copies, using a primer which contains a base sequence complementary to that of said oligomer and a part of recognition sequence of restriction sequence and which has selective sequences of 1 to 4 bases at the 3'-terminus to identity and select the base sequence at one terminus of the DNA fragment.

11. A sequencing method comprising the step of determining the base sequence by electrophoresis of sequencing reaction products using as a template DNA fragments obtained from the DNA preparation method according to any of Claims 1 to 10.

12. A DNA preparation method of obtaining the DNA fragment as a template for DNA base sequencing, said method comprising a process of DNA complementary strand extension covering the fragments digested by said different types of restriction enzymes by the steps of:
(1) digesting the sample DNAs by at least two different types of restriction enzymes (first and second restriction enzymes) to produce first and second fragment groups independently;
(2) mixing the first and the second groups after modification of the termini of the digested DNA fragments with the first and the second oligomers, respectively; and
(3) complementary strand extension using a primer to be hybridized with at least one modified terminus.

13. A DNA preparation method according to Claim 12 wherein the first oligomer having a known sequence is introduced into the 3'-terminus of the DNA fragment digested by at least one type of enzyme (first enzyme) to become part of the area to be hybridized with said primer.

14. A DNA preparation method according to Claim 12 or 13 further comprising:
(1) a step of adding the second oligomer having a sequence different from that of said first oligomer to the 5'-terminus of the DNA fragment pertaining to the second DNA fragment group; and
(2) a step of carrying out complementary strand extension using a first DNA primer having the sequence complementary with that of said first oligomer by 10 mer or more, and a second DNA probe (primer) having virtually the same sequence as that of said second oligomer by 10 mer or more.

15. A DNA preparation method according to any of Claims 12 to 14 further comprising:
(1) a step of blocking the strand extension capacity of the 3'-terminus of DNA fragment pertaining to said second DNA fragment group; and
(2) a step of allowing said second DNA probe to be hybridized only with the complementary strand extension of said first DNA probe.

16. A DNA preparation method for using complementary strand extension to produce DNA fragments covering said first and second DNA fragment groups, said method comprising:
(1) a step of producing a first DNA fragment group generated by digesting the sample DNA with a first restriction enzyme, and a second DNA fragment group generated by digesting the sample DNA with a second restriction enzyme;
(2) a step of mixing said first DNA fragment group with said second DNA fragment group; and
(3) a step of carrying out complementary strand extension at least once, using a first probe to be hybridized with said first DNA fragment group and a second probe to be hybridized with said second DNA fragment group.

17. A reagent kit used for DNA preparation to obtain the DNA fragments for DNA base sequencing, said kit comprising at least one restriction enzyme, at least 16 species of the DNA probes having a known sequence portion of 10 mer or more on the 5'-terminus, all or part of the restriction enzyme recognition sequences, and the selective sequence comprising two bases on the 3'-terminus, or virtually the same combinations thereof. the same combinations thereof.

18. A reagent kit according to Claim 17 wherein the restriction enzyme is a four-base recognition endonuclease.

19. A base sequence determination method in which determination of the base sequence is conducted by electrophoresis of sequencing reaction products using as a template the DNA fragments, obtained by the DNA preparation method according to any of Claims 12 to 16.

20. A base sequence determination method in which determination of the base sequence is conducted by electrophoresis of sequencing reaction products using as a template the DNA fragments obtained by the DNA preparation method using the reagent kit according to Claim 17 or Claim 18.
